# EUROPÄISCHE PATENTSCHRIFT

(11) **EP 1 389 192 B1**
(45) Veröffentlichungstag und Bekanntmachung des Hinweises auf die Patenterteilung: **12.04.2006**
(21) Anmeldenummer: 02769415.7
(22) Anmeldetag: 30.04.2002
(51) Int. Cl.: C07D 277/74, C09K 15/30, C23F 11/16

(54) **ETHERCARBONSÄUREN AUF BASIS VON ALKOXYLIERTER MERCAPTOBENZTHIAZOLE UND IHRE VERWENDUNG ALS KORROSIONSINHIBUTOREN**
ETHER CARBOXYLIC ACIDS BASED ON ALKOXYLATED MERCAPTOBENZOTHIAZOLE AND THE USE OF THE SAME AS CORROSION INHIBITORS
ACIDES ETHER CARBOXYLIQUES A BASE DE MERCAPTOBENZOTHIAZOLE ALCOXYLE ET LEUR UTILISATION COMME INHIBITEURS DE CORROSION

(30) Priorität: 12.05.2001 DE 10123210
(43) Veröffentlichungstag der Anmeldung: 18.02.2004
(73) Patentinhaber: Clariant Produkte (Deutschland) GmbH, 65929 Frankfurt am Main (DE)
(72) Erfinder: DAHLMANN, Uwe, 69126 Heidelberg (DE); FEUSTEL, Michael, 55278 Köngernheim (DE); KUPFER, Rainer, 65795 Hattersheim (DE)
(86) Internationale Anmeldenummer: PCT/EP2002/004730
(87) Internationale Veröffentlichungsnummer: WO 2002/092583

(56) Entgegenhaltungen:
- DE-A- 3 341 633
- US-A- 2 498 617

## Beschreibung

Die vorliegende Erfindung betrifft Ethercarbonsäuren aus alkoxylierten Mercaptobenzthiazolen sowie deren Verwendung als Metallbearbeitungshilfsmittel und Korrosionsinhibitor.

Ethercarbonsäuren, d.h. organische Carbonsäuren, die neben der Carboxylfunktion eine oder mehrere Etherbrücken tragen, bzw. deren Alkali- oder Aminsalze, sind als milde Detergenzien mit hohem Kalkseifendispergiervermögen bekannt. Sie finden sowohl in Waschmittel- und Kosmetikformulierungen, aber auch in technischen Anwendungen (z.B. Metallbearbeitungsflüssigkeiten, Kühlschmiermittel) Verwendung. Diese Produkte werden gemäß dem Stand der Technik entweder durch Alkylierung von Alkohol- oder Fettalkoholoxethylaten oder -oxpropylaten mit Chloressigsäurederivaten (Williamsonsche Ethersynthese) oder aus den gleichen Ausgangsprodukten durch Oxidation mit verschiedenen Reagenzien (Luftsauerstoff, Hypochlorit, Chlorit) unter Katalyse mit verschiedenen Katalysatoren hergestellt.

DE-A-199 28 128 offenbart ein Verfahren zur Herstellung von Ethercarbonsäuren mit niedrigem Restalkoholgehalt, indem Fettalkohole zunächst unter Einsatz nichtkatalytischer Mengen an Alkalikatalysator (NaOH, KOH, Alkoholate über 5 Mol-%) mit Alkylenoxiden umgesetzt werden, und die resultierenden hochalkalischen Reaktionsmischungen, die aus einem Gemisch von oxethylierten Alkoholen und Alkoholaten verschiedener Polyalkylenglykolether bestehen, anschließend in einer klassischen Williamson-Synthese mit Natriumchloracetat in die entsprechende Ethercarbonsäure überführt werden. Hierdurch wird der Restgehalt an Fettalkohol in der Ethercarbonsäure ohne spezielle Katalysatoren verringert.

US-4.568,753 offenbart eine rosthemmende Substanz, enthaltend eine Verbindung der allgemeinen Formel 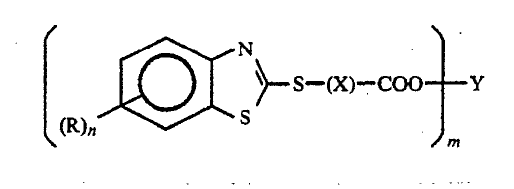 worin
- R: eine Amino-, Methyl-, Chlor-, Carboxy- oder Hydroxyl-Gruppe ist;
- n: eine gerade Zahl zwischen 0 und 2 ist;
- X: ein gerades oder verzweigtes, divalentes aliphatisches Hydrocarbon-Radikal mit 0 bis 5 Kohlenstoffatomen oder ein divalentes aromatisches Hydrocarbon-Radikal mit 6 - 10 Kohlenstoffatomen ist;
- Y: gleich Wasserstoff, ein Alkalimetall, ein Erdalkalimetall, Ammonium oder eine substituierte Ammoniumgruppe ist; und
- m: eine gerade Zahl ist, deren Wert gleich der Valenzzahl ist.

GB-A-2 319 530 offenbart einen Korrosionsinhibitor für Eisenmetalle, der insbesondere für die Ölförderung geeignet ist. Dieser umfasst eine geradkettige oder verzweigte Mercaptocarbonsäure mit 2 bis 6 Kohlenstoffatomen, und ein mit Carbonyl- und Carboxylgruppen substituiertes Amin.

US-6 117 364 offenbart einen Korrosionsinhibitor, der gegen Säurekorrosion an Geräten wirkt, die in der Erdölförderung Anwendung finden. Er umfasst eine Mischung aus Zimtaldehyd und organischen Schwefelverbindungen, unter anderem 2-Mercaptobenzthiazol.

Mercaptobenzthiazol und verschiedene seiner Derivate, sowie Verfahren zu ihrer Herstellung sind im Stand der Technik beschrieben.

US-2 498 617 offenbart Mercaptobenzthiazole, die mit bis zu 30 Mol Ethylenoxid ethoxyliert sind. Verbindungen dieser Art werden als Inhibitoren bei der sauren Abbeizung von Eisen und Stahl verwendet.

US-2 695 299 und US-2 762 786 offenbaren Verbindungen der Formel 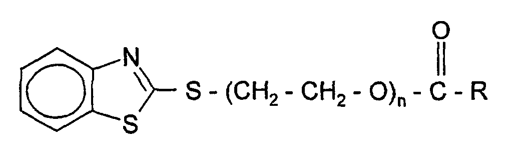 worin n eine Zahl von 1 bis 30 und R einen Kohlenwasserstoffrest bedeuten. Verbindungen dieser Art werden als Weichmacher für Kunststoffe bezeichnet.

Die Eigenschaften von Stoffen, die als korrosionsinhibierende Mittel zur Metallbearbeitung als auch für Erdöl- und Erdgasförderung und -verarbeitung verwendet werden, hängen stark von deren Fähigkeit ab, gut haftende Filme auf Metalloberflächen zu bilden. Diese Filme sollen auch bei starker mechanischer Belastung, wie beim Schleifen, Schneiden und Bohren von Metallwerkstücken bzw. bei hohen Fließgeschwindigkeiten in Erdgas-/Erdölpipelines, persistent sein. Es hat sich gezeigt, dass die Ethercarbonsäuren des Standes der Technik nicht in allen Fällen eine ausreichende Filmbildung und Persistenz aufzeigen, und dadurch keinen ausreichenden Korrosionsschutz bieten.

Es bestand daher die Aufgabe, neue Stoffe aufzufinden, die eine verbesserte Filmbildung und Filmpersistenz zeigen.

Überraschenderweise wurde gefunden, dass Ethercarbonsäuren aus Mercaptobenzthiazolen ausgezeichnete Filmbildung und verbesserten Korrosionsschutz aufweisen.

Gegenstand der Erfindung sind daher Verbindungen der Formel (1) 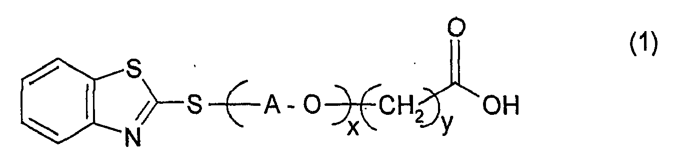 sowie deren Salze der Formel 2 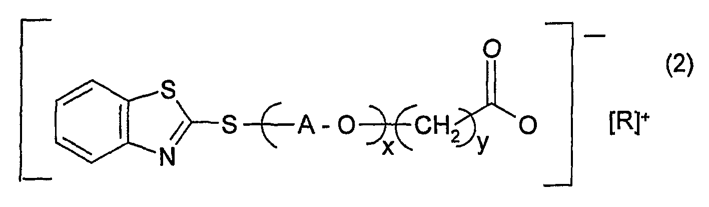 worin
- A: C₂- bis C₄-Alkylen,
- x: eine Zahl von 1 bis 100, und
- y: eine Zahl von 1 bis 4, und
- R: ein Kation
bedeuten.

Ein weiterer Gegenstand der Erfindung ist die Verwendung der Verbindungen der Formel 1 und/oder 2, vorzugsweise Verbindungen der Formel 1, als Korrosionsinhibitor, vorzugsweise in der Erdöl- und Erdgasförderung und -verarbeitung.

Ein weiterer Gegenstand der Erfindung ist die Verwendung der Verbindungen der Formel 1 und/oder 2 als Metallbearbeitungsmittel.

A bedeutet vorzugsweise Propylen oder Ethylen, insbesondere Ethylen. In einer weiteren bevorzugten Ausführungsform der Erfindung steht die Gruppe -(A-O)ₓ₋für eine gemischte Alkoxygruppe, die Ethylen-, Propylen- und Butylenreste enthalten kann. Handelt es sch um eine gemischte Alkoxygruppe, so liegt das Verhältnis vom Ethylenoxid abgeleiteten Gruppen zu den von Propylen- oder Butylenoxid abgeleiteten Gruppen vorzugsweise zwischen 10:1 und 1:1.

x steht vorzugsweise für eine Zahl zwischen 2 und 70, insbesondere 3 bis 50.

y steht vorzugsweise für eine Zahl von 1 oder 2, insbesondere 1.

R kann in einer bevorzugten Ausführungsform für Wasserstoffionen stehen. In einer weiteren bevorzugten Ausführungsform steht R für Alkali- oder Erdalkaliionen, insbesondere Lithium, Natrium, Kalium, Magnesium, oder Calcium.

In einer weiteren bevorzugten Ausführungsform werden als Kationen Ammoniumionen der Formel NR¹R²R³R⁴ verwendet, wobei R¹, R², R³ und R⁴ unabhängig voneinander H, C₁- bis C₂₂-Alkyl, C₆- bis C₁₈-Aryl, C₇- bis C₂₂-Alkylaryl und/oder C₁- bis C₂₂-Alkenyl bedeuten können. Die Reste R¹, R², R³ und R⁴ können Heteroatome wie N, P, O, S enthalten. Die Ammoniumreste können Monoalkylammonium-, Dialkylammonium-, Trialkylammonium- oder Tetraalkylammoniumreste sein, worin die Alkylsubstituenten unabhängig voneinander mit bis zu 3 Hydroxygruppen besetzt sein können. Vorzugsweise steht R für Ammoniumreste, die einen, zwei, drei oder vier C₂- bis C₁₀-Alkylreste tragen. In einer weiteren bevorzugten Ausführungsform können einer, zwei oder drei der Reste R¹ bis R⁴ alkoxyliert sein.

Geeignete Amine für die Herstellung von Ammoniumkationen R sind Monoamine mit primärer oder sekundärer Aminofunktion wie Methylamin, Ethylamin, Butylamin, Laurylamin, Cocosfettamin, Stearylamin, Dimethylamin, Diethylamin, Dibutylamin, aber auch Di- und Polyamine wie z.B. 3-Dimethylaminopropylamin, 3-Diethylaminopropylamin, 3-Morpholinopropylamin, Diethylentriamin, Triethylentetramin oder Tetraethylenpentamin.

Geeignete Aminoalkohole für die Herstellung von Ammoniumkationen R sind beispielsweise N,N-Dimethylaminoethanol, N,N-Diethylaminoethanol, N,N-Dibutylaminoethanol, 3-Dimethylaminopropanol, N-Hydroxyethylmorpholin, Monoethanolamin, Diethanolamin, Triethanolamin, 3-Aminopropanol, lsopropanolamin, 2(2-Aminoethoxy)ethanol und Cyclohexylamino- N,N-diethanol.

Geeignete Aminalkylthiole für die Herstellung von Ammoniumkationen R sind Cysteamin und Cystamin.

Die erfindungsgemäßen Verbindungen können dadurch hergestellt werden, dass man Mercaptobenzthiazol zunächst alkoxyliert und dann mit Monochlorcarbonsäuren umsetzt. Mercaptobenzthiazol wird im folgenden mit MBT bezeichnet.

MBT wird im allgemeinen mit Ethylenoxid, Propylenoxid, Butylenoxid oder Mischungen verschiedener solcher Alkylenoxide umgesetzt, wobei Ethylenoxid oder Mischungen aus Ethylenoxid und Propylenoxid bevorzugt sind. Bezogen auf MBT werden 1-30 Mol Alkylenoxid beaufschlagt, bevorzugt 1-12 Mol.

Geeignete Lösemittel für die Alkoxylierung sind inerte Ether wie Dioxan, Tetrahydrofuran, Glyme, Diglyme und MPEGs. Wasser als auch Alkohole, wie Propanole, Butanole sowie oxethylierte Monoalkohole wie Butylglykol, Isobutylglykol und Butyldiglykol können zur Anwendung kommen, führen aber zu einem hohen Anteil an Nebenprodukten.

Als basische Verbindung zur Herstellung des oxethylierten MBT können Erdalkali-/Alkalimetallhydroxide oder-alkoholate (Natriummethylat, Natriumethylat, Kalium-tert.-butylat) verwendet werden, bevorzugt sind aber Alkalimetallhydroxide, besonders Natriumhydroxid oder Kaliumhydroxid.

Die basischen Verbindungen werden in Mengen von ca. 5-95 Mol-% bezogen auf MBT eingesetzt, bevorzugt zwischen 15 und 90 Mol-%, besonders bevorzugt zwischen 20-60 Mol-%.

Ausgehend vom MBT werden das zur Oxalkylierung notwendige Thiolat durch Umsetzung mit den basischen Verbindungen hergestellt. Um höhere Anteile an Nebenprodukten (Glykole, Glykolether niederer Alkohole) im Endprodukt zu vermeiden, sollte das hierbei entstehende Reaktionswasser oder der entsprechende niedere Alkohol aus dem Reaktionsgemisch vor der Umsetzung mit dem Alkylenoxid entfernt werden. Dies kann entweder durch Umsetzung des MBT mit einem Alkalihydroxid und Abdestillieren des Reaktionswassers geschehen oder durch Umsetzung des Basisalkohols mit einem Alkoholat eines niederen Alkohols und Abdestillieren des niederen Alkohols. Andererseits kann MBT in einem Zweischrittverfahren, im ersten Schritt ohne Zugabe der basischen Verbindungen monoalkoxyliert werden. In einem weiteren Schritt erfolgt dann die notwendige Umsetzung zum Alkoholat.

Die erhaltene Mischung aus MBT und dem entsprechenden Thiolat bzw. Alkoholat wird dann mit ca. 1-30 mol eines Alkylenoxids, bevorzugt Ethylenoxid und/oder Propylenoxid umgesetzt, die Reaktionstemperaturen liegen hierbei bei ca. 80 bis 160°C. Dabei tritt bei einer mit höheren Alkalimengen katalysierten Reaktion eine engere Homologenverteilung ein.

Im anschließenden Reaktionsschritt wird die MBT-Oxethylat-Mischung mit einem Chlorcarbonsäurederivat und einer Base, bevorzugt trockenem Chloressigsäure-Natriumsalz und Natriumhydroxid umgesetzt. Dies kann geschehen, indem man die Oxethylat-Mischung mit 100 bis 150 Mol-% Natriumchloracetat bei 30 bis 100°C umsetzt und gleichzeitig oder nacheinander mit festem Natriumhydroxid oder Kaliumhydroxid versetzt, so dass die Summe aus der in der Oxethylatmischung bereits vorliegenden Base und der zusätzlich zugegebenen Basenmenge der Menge an Natriumchloracetat entspricht. Die aus der Umsetzung mit dem Alkylenoxid bereits enthaltene Basenmenge kann somit direkt für die anschließende Williamson-Synthese genutzt werden und muss nicht, wie bei der Synthese eines Standard-Oxethylats, ausgewaschen werden.

Anschließend an die Alkylierungsreaktion kann die entstehende Lösung des MBT-Ethercarbonsäure-Alkalisalzes entweder direkt als erfindungsgemäße Verbindung Verwendung finden oder in die freie MBT-Ethercarbonsäure überführt werden. Hierzu wird mit starker Mineralsäure (Salzsäure, Schwefelsäure) auf pH < 3 angesäuert und die MBT-Ethercarbonsäure durch Phasentrennung oberhalb ihres Trübungspunktes heiß als Oberphase abgetrennt.

Die Herstellung der erfindungsgemäßen MBT-Ethercarbonsäureammoniumsalze erfolgt im allgemeinen durch direkte Umsetzung der freien Säure mit den entsprechend funktionalisierten Aminen bei Temperaturen unter 60 °C.

### Beispiele:

### Beispiel 1 (MBT + 1EO):

In einem 2 l Ethoxylierungsautoklaven wurden 700 g MBT in Dioxan (1:1) unter Stickstoffspülung suspendiert und die Reaktionsmischung auf 120-130 °C aufgeheizt. Dann wurde ohne Katalysatorzugabe bis zur Druckkonstanz Ethylenoxid (EO) aufgepresst und 1 h bei 150°C nachreagiert. Nach Gewichtsbilanz ergab sich eine EO-Aufnahme von 1 mol EO/mol MBT. Das Dioxan wurde zu 90 % durch Abdestillation entfernt und als Zwischenprodukt wurde MBT + 1EO in Restdioxan als trübe viskose Flüssigkeit erhalten.

### Beispiel 2 (MBT + 3 EO)

In einem 2 l Ethoxylierungsautoklaven wurden 950 g Zwischenprodukt MBT + 1 EO unter Stickstoffspülung vorgelegt und unter NaOH Katalyse (1 %) bei 120 bis 130°C mit Ethylenoxid begast, bis 2 mol EO unter Druckkonstanz abreagiert waren. Es wurde 1 h bei 150°C nachreagiert. Nach Abdestillation leicht flüchtiger Komponenten wurde das Produkt MBT + 3EO als hellbraune viskose Flüssigkeit erhalten. Nach OH-Zahl ergab sich ein durchschnittlicher EO-Gehalt von 2,7.

### Beispiel 3 (MBT + 5 EO)

In einem 2 l Ethoxylierungsautoklaven wurden 950 g Zwischenprodukt MBT + 1 EO unter Stickstoffspülung vorgelegt und unter NaOH Katalyse (1 %) bei 120 bis 130°C mit Ethylenoxid begast, bis 4 mol EO unter Druckkonstanz abreagiert waren. Es wurde 1 h bei 150°C nachreagiert. Nach Abdestillation leicht flüchtiger Komponenten wurde das Produkt MBT + 5EO als rotbraune viskose Flüssigkeit erhalten. Nach OH-Zahl ergab sich ein durchschnittlicher EO-Gehalt von 4,9.

### Beispiel 4 (MBT + 3 PO + 3 EO)

In einem 2 l Ethoxylierungsautoklaven wurden 700 g MBT in Dioxan (1:1) unter Stickstoffspülung vorgelegt und unter NaOH-Katalyse (1 %) bei 120 bis 130°C mit Propylenoxid (PO) begast, bis 3 mol PO unter Druckkonstanz abreagiert waren. Es wurde 1 h bei 150°C nachreagiert. Nach OH-Zahl einer entnommenen Probe ergab sich ein durchschnittlicher PO-Gehalt von 3,5 mol. Nach Abdestillation von Dioxan und leicht flüchtiger Komponenten wurde das so erhaltene MBT + 3 PO bei 120 bis 130°C weiter mit Ethylenoxid beaufschlagt, bis 3 mol EO unter Druckkonstanz abreagiert waren. Es wurde 1 h bei 150°C nachreagiert. Nach erneuter Abdestillation leicht flüchtiger Komponenten wurde das Produkt MBT 3 PO + 3EO als braune viskose Flüssigkeit erhalten. Nach OH-Zahl ergab sich ein durchschnittlicher EO-Gehalt von 3,0 mol.

### Beispiel 5 (MBT + 3 PO + 7 EO)

In einem 2 l Ethoxylierungsautoklaven wurden 700 g MBT in Dioxan (1:1) unter Stickstoffspülung vorgelegt und unter NaOH-Katalyse (1 %) bei 120 bis 130°C mit Propylenoxid begast, bis 3 mol PO unter Druckkonstanz abreagiert waren. Es wurde 1 h bei 150°C nachreagiert. Nach OH-Zahl einer entnommenen Probe ergab sich ein durchschnittlicher PO-Gehalt von 3,6 mol. Nach Abdestillation von Dioxan und leicht flüchtiger Komponenten wurde das so erhaltene MBT + 3 PO bei 120 bis 130°C weiter mit Ethylenoxid beaufschlagt, bis 7 mol EO unter Druckkonstanz abreagiert waren. Es wurde 1 h bei 150°C nachreagiert. Nach erneuter Abdestillation leicht flüchtiger Komponenten wurde das Produkt MBT 3 PO + 7EO als braune viskose Flüssigkeit erhalten. Nach OH-Zahl ergab sich ein durchschnittlicher EO-Gehalt von 6,9 mol.

### Beispiel 6 (MBT + 4 PO + 7 EO)

In einem 2 l Ethoxylierungsautoklaven wurden 700 g MBT in Dioxan (1:1) unter Stickstoffspülung vorgelegt und unter NaOH-Katalyse (1 %) bei 120 bis 130°C mit Propylenoxid begast, bis 4 mol PO unter Druckkonstanz abreagiert waren. Es wurde 1 h bei 150°C nachreagiert. Nach OH-Zahl einer entnommenen Probe ergab sich ein durchschnittlicher PO-Gehalt von 4,9 mol. Nach Abdestillation von Dioxan und leicht flüchtiger Komponenten wurde das so erhaltene MBT + 3 PO 120 bis 130°C weiter mit Ethylenoxid beaufschlagt, bis 7 mol EO unter Druckkonstanz abreagiert waren. Es wurde 1 h bei 150°C nachreagiert. Nach erneuter Abdestillation leicht flüchtiger Komponenten wurde das Produkt MBT 3 PO + 6EO als braune viskose Flüssigkeit erhalten. Nach OH-Zahl ergab sich ein durchschnittlicher EO-Gehalt von 7,2 mol.

### Beispiel 7 (MBT + 3 EO-ECS)

In einer 2 l Rührapparatur wurden 572 g MBT + 3 EO (2 mol entsprechend OH-Zahl) unter Stickstoffspülung vorgelegt und auf 40°C erwärmt. Dann wurden 325 g (2,4 mol) Natriumchloracetat eingetragen und die Reaktionsmischung auf 50°C erwärmt. Nach jeweils 30 min wurden 96 g (2,4 mol) NaOH-Microprills in 6 Portionen so zugegeben, dass die Temperatur 55°C nicht übersteigt. Es wurde 2 h bei 70°C nachreagiert. Danach wurde 900 g 10 % Salzsäure zulaufen lassen, die Mischung auf 95°C erhitzt und in eine beheizbare Rührapparatur mit Bodenablass überführt. Die Phasentrennung erfolgte nach 15 min bei 105°C, wobei 1244 g wässrige Unterphase abgetrennt und 647 g MBT + 3 EO-ECS erhalten wurden.

### Beispiel 8 (MBT + 5 EO-ECS)

In einem 2 l Rührapparatur wurden 575 g MBT + 5 EO (1,5 mol entsprechend OH-Zahl) unter Stickstoffspülung vorgelegt und auf 40°C erwärmt. Dann wurden 244 g (1,8 mol) Natriumchloracetat eingetragen und die Reaktionsmischung auf 50°C erwärmt. Nach jeweils 30 min wurden 72 g (1,8 mol) NaOH-Microprills in 4 Portionen so zugegeben, dass die Temperatur 55°C nicht übersteigt. Es wurde 2 h bei 70°C nachreagiert. Danach wurde 675 g 10 % Salzsäure zulaufen lassen, die Mischung auf 95°C erhitzt und in eine beheizbare Rührapparatur mit Bodenablass überführt. Die Phasentrennung erfolgte nach 15 min bei 105°C, wobei.909 g wässrige Unterphase abgetrennt und 640 g MBT + 5 EO-ECS erhalten wurden.

### Beispiel 9 (MBT+3 PO + 3 EO-ECS)

In einem 2 l Rührapparatur wurden 754 g MBT + 3 PO + 3 EO (1,5 mol entsprechend OH-Zahl) unter Stickstoffspülung vorgelegt und auf 40°C erwärmt. Dann wurden 244 g (1,8 mol) Natriumchloracetat eingetragen und die Reaktionsmischung auf 50°C erwärmt. Nach jeweils 30 min wurden 72 g (1,8 mol) NaOH-Microprills in 4 Portionen so zugegeben, dass die Temperatur 55°C nicht übersteigt. Es wurde 2 h bei 70°C nachreagiert. Danach wurde 675 g 10 % Salzsäure zulaufen lassen, die Mischung auf 95°C erhitzt und in eine beheizbare Rührapparatur mit Bodenablass überführt. Die Phasentrennung erfolgte nach 15 min bei 105°C, wobei 919 g wässrige Unterphase abgetrennt und 820 g MBT + 3 PO + 3 EO-ECS erhalten wurden.

### Beispiel 10 (MBT + 3 PO + 7 EO-ECS)

In einem 2 l Rührapparatur wurden 687 g MBT +3 PO + 7 EO (1 mol entsprechend OH-Zahl) unter Stickstoffspülung vorgelegt und auf 40°C erwärmt. Dann wurden 163 g (1,2 mol) Natriumchloracetat eingetragen und die Reaktionsmischung auf 50°C erwärmt. Nach jeweils 30 min wurden 48 g (1,2 mol) NaOH-Microprills in 4 Portionen so zugegeben, dass die Temperatur 55°C nicht übersteigt. Es wurde 2 h bei 70°C nachreagiert. Danach wurde 450 g 10 % Salzsäure zulaufen lassen, die Mischung auf 95°C erhitzt und in eine beheizbare Rührapparatur mit Bodenablass überführt. Die Phasentrennung erfolgte nach 1h bei 105°C, wobei 627 g wässrige Unterphase abgetrennt und 708 g MBT + 3 PO + 7 EO-ECS erhalten wurden.

### Beispiel 11 (MBT + 4 PO + 7 EO-ECS)

In einem 2 l Rührapparatur wurden 749 g MBT + 4 PO + 7 EO (1 mol entsprechend OH-Zahl) unter Stickstoffspülung vorgelegt und auf 40°C erwärmt. Dann wurden 163 g (1,2 mol) Natriumchloracetat eingetragen und die Reaktionsmischung auf 50°C erwärmt. Nach jeweils 30 min wurden 48 g (1,2 mol) NaOH-Microprills in 4 Portionen so zugegeben, dass die Temperatur 55°C nicht übersteigt. Es wurde 2 h bei 70°C nachreagiert. Danach wurde 450 g 10 % Salzsäure zulaufen lassen, die Mischung auf 95°C erhitzt und in eine beheizbare Rührapparatur mit Bodenablass überführt. Die Phasentrennung erfolgte nach 1h bei 105°C, wobei 632 g wässrige Unterphase abgetrennt und 771 g MBT + 3 PO + 7 EO-ECS erhalten wurden.

### Beispiel 12:

150 g MBT + 3 EO-ECS aus Beispiel 7 wurde bei 40 °C und kontinuierlichen Rühren mit 1 N NaOH unter Bildung des entsprechenden Salzes auf pH 7,8 eingestellt.

### Beispiel 13:

150 g MBT + 3 EO-ECS aus Beispiel 7 wurde bei 40 °C und kontinuierlichen Rühren mit Monoethanolamin (MEA) unter Bildung des entsprechenden Ammoniumsalzes auf pH 10,2 eingestellt.

### Beispiel 14:

150 g MBT + 3 EO-ECS aus Beispiel 7 wurde bei 40 °C und kontinuierlichen Rühren mit Triethanolamin (TEA) unter Bildung des entsprechenden Ammoniumsalzes auf pH 8,9 eingestellt.

### Beispiel 15:

150 g MBT + 3 EO-ECS aus Beispiel 7 wurde bei 40 °C und kontinuierlichen Rühren mit 1-Amino-2-propanol (MIPA) unter Bildung des entsprechenden Ammoniumsalzes auf pH 9,8 eingestellt.

### Beispiel 16:

150 g MBT + 3 EO-ECS aus Beispiel 7 wurde bei 40 °C und kontinuierlichen Rühren mit Cyclohexylamin unter Bildung des entsprechenden Ammoniumsalzes auf pH 9,8 eingestellt.

### Beispiel 17:

150 g MBT + 5 EO-ECS aus Beispiel 8 wurde bei 40 °C und kontinuierlichen Rühren mit Triethanolamin (TEA) unter Bildung des entsprechenden Ammoniumsalzes auf pH 8,9 eingestellt.

### Beispiel 18:

150 g MBT + 3 PO + 3 EO-ECS aus Beispiel 9 wurde bei 40 °C und kontinuierlichen Rühren mit Triethanolamin (TEA) unter Bildung des entsprechenden Ammoniumsalzes auf pH 8,9 eingestellt.

Verwendung der erfindungsgemäßen Verbindungen als Korrosionsinhibitor für wassermischbare Kühlschmiermittel, Reinigungsflüssigkeiten, Oberflächenbehandlungen.

Die Korrosionsschutzprüfung erfolgte in Anlehnung an die DIN-Norm 51360, Teil 2 (Filterpapiertest) und dient der Beurteilung der Korrosion von Eisenmetall. Als Maß für die Korrosion dienen Art und Anzahl der Korrosionsabzeichnungen auf einem Rundfilter, die durch Einwirken eines mit Wasser gemischten Kühlschmierstoffes (KSS) auf genormte Graugussspäne (Spangröße: 3 bis 6 mm²) entstehen. Die Beurteilung erfolgt durch Sichtprüfung und Einstufung des Korrosionsgrades (1 bis 4) nach Vergleichstabelle.

Als Vergleich wurden kommerziell erhältliche Emulgatoren (Emulsogen® COL 020 und COL 050) herangezogen.

Es handelt sich dabei im wesentlichen um Ethercarbonsäuren der Zusammensetzung Oleyl-O-(EO)₂-CH₂-COOH (Emulsogen COL 020) bzw. das Homologe mit 5 EO-Gruppen (Emulsogen COL 050). Diese wurden mit Triethanolamin (TEA) unter Bildung des entsprechenden Ammoniumsalzes auf pH 8,9 eingestellt.

**Tabelle 1: Korrosionsschutzprüfung nach DIN (Filterpapiertest), Angabe in Korrosionsgraden 1 bis 4 nach Vergleichstabelle DIN-Norm 51360, Teil 2 (Filterpapiertest), Konzentrationen in Gew.-%**

| Beispiel | Korrosionsinhibitor/ Passivator | Konzentration des Korrosionsinhibitors/Passivators | | |
|---|---|---|---|---|
| | | 2 % | 3 % | 4 % |
| 19 (V) | Emulsogen COL 020 | - | 0-1 | 0 |
| 20 (V) | Emulsogen COL 050 | 1-2 | 0-1 | 0 |
| 21 | aus Beispiel 12 | 3 | 2 | 1 |
| 22 | aus Beispiel 13 | 0-1 | 0 | 0 |
| 23 | aus Beispiel 14 | 2 | 0-1 | 0 |
| 24 | aus Beispiel 15 | 1-2 | 0-1 | 0 |
| 25 | aus Beispiel 16 | 1-2 | 0-1 | 0 |
| 26 | aus Beispiel 17 | 2 | 0-1 | 0 |
| 27 | aus Beispiel 18 | 2 | 0-1 | 0 |

### Kupferstreifentest:

Der Kupferstreifentest dient der optischen Begutachtung der Oberflächenqualität von Nichteisenmetallen (VKIS-Methode, Arbeitsblatt 7). Zur Vorbehandlung der Kupferstreifen erhitzt man diese bis zur Rotglut und schreckt in Methanol ab. Der so gereinigte Kupferstreifen wird in einen 100 ml Standzylinder gegeben, der mit einer 4 %igen Emulsion des zu prüfenden KSS in E-Wasser gefüllt ist. Nach 14-tägiger Lagerung bei 50°C werden die Kupferstreifen aus den Emulsionen genommen und durch Sichtprüfung beurteilt.

### Kupferpulvertest:

In einen 100 ml Standzylinder werden eine 4 %ige Emulsion des zu prüfenden KSS in E-Wasser und exakt 1 g Kupferpulver gegeben. Der Passivator wurde zu 3 % in den KSS einformuliert. Nach 14-tägiger Lagerung bei 50°C werden die Emulsionen vom Kupferpulver abfiltriert und der Gehalt an Cu^{+/2+} analysiert.

Zum Vergleich wurde kommerziell erhältliches Benzotriazol (Irgamet® BT) und Mercaptobenzothiazolylessigsäure als Standardpassivator für Kupfer herangezogen.

**Tabelle 2: Kupferstreifentest (nach VKIS) und Kupferpulvertest**

| Beispiel | Korrosionsinhibitor/ Passivator | pH-Wert | Kupferstreifentest | Kupferpulvertest µg Cu/ml |
|---|---|---|---|---|
| 28 (V) | ohne | 9,1 | starke Verfärbung | 0,44 |
| 29 (V) | Irgamet BT | 9,1 | unverändert | 0,18 |
| 30 (V) | Mercaptobenzothiazolylessigsäure | 9,1 | unverändert | 0,43 |
| 31 | aus Beispiel 7 | 9,1 | unverändert | 0,10 |
| 32 | aus Beispiel 8 | 9,1 | leichte Verfärbung | 0,11 |
| 33 | aus Beispiel 9 | 9,1 | unverändert | 0,10 |

Verwendung der erfindungsgemäßen Verbindungen als Korrosionsinhibitor für Exploration, Produktion und Raffinerie von Erdölen und Erdgasen

Die Korrosionsinhibitoren wurden im Shell-wheel-test geprüft. Coupons aus C-Stahl (DIN 1.1203 mit 15 cm² Oberfläche) wurden in eine Salzwasser/Petroleum-Mischung (9:1,5 %ige NaCl-Lösung mit Essigsäure auf pH 3,5 gestellt) eingetaucht und bei einer Umlaufgeschwindigkeit von 40 rpm bei 70°C 24 Stunden diesem Medium ausgesetzt. Die Dosierung des Inhibitors betrug 50 ppm einer 40 % Lösung des Inhibitors. Die Schutzwerte wurden aus der Massenabnahme der Coupons, bezogen auf einen Blindwert, berechnet.

Thioglycolsäure ist eine Verbindung der Formel HS-CH₂-COOH.

**Tabelle 3: (SHELL-Wheel-Test)**

| Beispiel | Korrosionsinhibitor | Schutz % |
|---|---|---|
| 34 (V) | Thioglycolsäure | 70-74 |
| 35 | aus Beispiel 7 | 86-90 |
| 36 | aus Beispiel 8 | 80-85 |
| 37 | aus Beispiel 9 | 90-94 |
| 38 | aus Beispiel 10 | 82-87 |
| 38 | aus Beispiel 11 | 85-90 |

**Tabelle 4: (LPR-Test)**

| Beispiel | Korrosionsinhibitor | Schutz nach [%] | | |
|---|---|---|---|---|
| | | 10 min | 30 min | 60 min |
| 39 (V) | Thioglycolsäure | 8,4 | 56,3 | 84,3 |
| 40 | aus Beispiel 7 | 90,9 | 95,3 | 96,2 |
| 41 | aus Beispiel 8 | 85,7 | 92,1 | 94,1 |
| 42 | aus Beispiel 9 | 90,6 | 96,2 | 97,4 |
| 43 | aus Beispiel 10 | 82,1 | 89,6 | 92,3 |
| 44 | aus Beispiel 11 | 89,2 | 93,4 | 96,8 |

## Patentansprüche

1. Verbindungen der Formel (1) 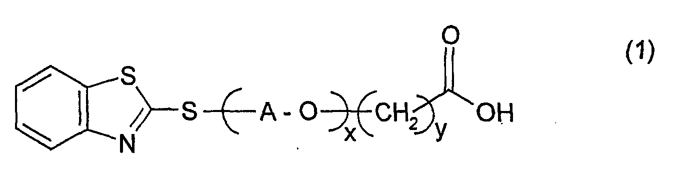 sowie deren Salze der Formel 2 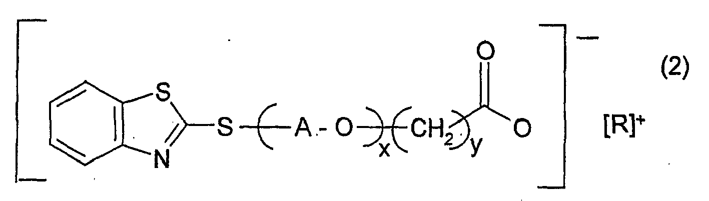 worin
A C₂- bis C₄-Alkylen,
x eine Zahl von 1 bis 100, und
y eine Zahl von 1 bis 4, und
R ein Kation
bedeuten.

2. Verbindungen gemäß Anspruch 1, worin A für Propylen und/oder Ethylen steht.

3. Verbindungen gemäß Anspruch 1 und/oder 2, worin x für eine Zahl zwischen 2 und 70 steht.

4. Verbindungen gemäß einem oder mehreren der Ansprüche 1 bis 3, worin R für Wasserstoff, ein Alkalimetallion, Erdalkalimetallion oder ein ggf. substituiertes Ammoniumion steht.

5. Verbindungen gemäß einem oder mehreren der Ansprüche 1 bis 4, worin y für 1 oder 2 steht.

6. Verwendung von Verbindungen gemäß einem oder mehreren der Ansprüche 1 bis 5 als Korrosionsinhibitor oder Metallbearbeitungsmittel.

7. Verwendung von Verbindungen gemäß Formel 1 nach einem oder mehrere des Ansprüche 1 bis 5 als Korrosionsinhibitor in der Erdöl- und Erdgasförderung und -verarbeitung.

## Claims

1. A compound of the formula (1) 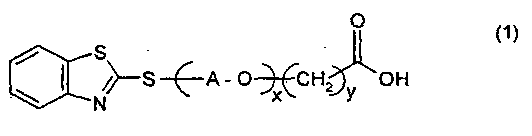 and salts thereof of the formula 2 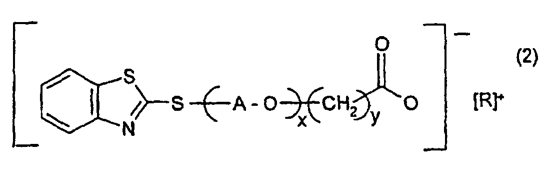 in which
A is C₂- to C₄-alkylene,
x is a number from 1 to 100, and
y is a number from 1 to 4, and
R is a cation.

2. The compound as claimed in claim 1, in which A is propylene and/or ethylene.

3. The compound as claimed in claim 1 and/or 2, in which x is a number between 2 and 70.

4. The compound as claimed in one or more of claims 1 to 3, in which R is hydrogen, an alkali metal ion, alkaline earth metal ion or an optionally substituted ammonium ion.

5. The compound as claimed in one or more of claims 1 to 4, in which y is 1 or 2.

6. The use of compounds as claimed in one or more of claims 1 to 5 as corrosion inhibitor or metal-working medium.

7. The use of compounds according to formula 1 and as claimed in one or more of claims 1 to 5 as corrosion inhibitor in crude oil and natural gas recovery and processing.

## Revendications

1. Composés de formule (1) 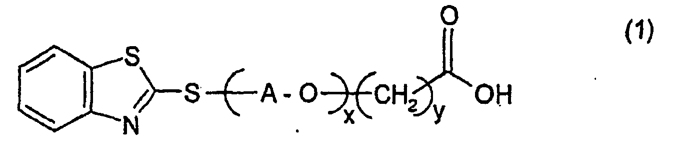 ainsi que leurs sels de formule (2) 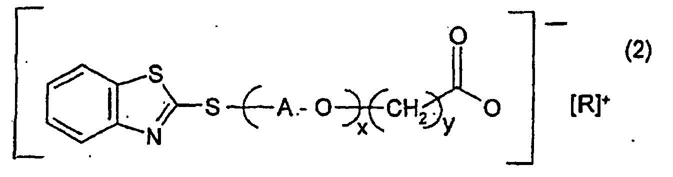 dans lesquelles
A est un radical alkylène en C₂ à C₄,
x est un nombre de 1 à 100, et
y est un nombre de 1 à 4, et
R est un cation.

2. Composés selon la revendication 1, dans lesquels A est le propylène et/ou l'éthylène.

3. Composés selon la revendication 1 et/ou 2, dans lesquels x est un nombre compris entre 2 et 70.

4. Composés selon l'une ou plusieurs des revendications 1 à 3, dans lesquels R est un atome d'hydrogène, un ion d'un métal alcalin, un ion d'un métal alcalino-terreux ou un ion ammonium éventuellement substitué.

5. Composés selon l'une ou plusieurs des revendications 1 à 4, dans lesquels y vaut 1 ou 2.

6. Utilisation de composés selon l'une ou plusieurs des revendications 1 à 5 en tant qu'inhibiteurs de corrosion ou agents d'usinage de métaux.

7. Utilisation de composés de formule (1) selon l'une ou plusieurs des revendications 1 à 5 en tant qu'inhibiteurs de corrosion lors de la récupération et du traitement du pétrole et du gaz naturel.
